# FASCICULE DE BREVET EUROPEEN

(11) **EP 1 267 801 B1**
(45) Date de publication et mention de la délivrance du brevet: **31.12.2008**
(21) Numéro de dépôt: 01921438.6
(22) Date de dépôt: 29.03.2001
(51) Int. Cl.: A61Q 5/06, A61K 8/22, A61K 8/26, A61K 8/29, A61K 8/27, A61K 8/58

(54) **COMPOSITION CONSMETIQUE A BASE DE NANOPARTICULES ET DE COMPOSES ORGANIQUES DU SILICIUM SOLUBLES DANS L'EAU.**
KOSMETISCHE ZUSAMMENSETZUNG AUF BASIS VON NANOPARTIKELN UND WASSERLÖSLICHEN ORGANOSILIZIUMVERBINDUNGEN
COSMETIC COMPOSITION BASED ON NANOPARTICLES AND WATER-SOLUBLE ORGANIC SILICON COMPOUNDS

(30) Priorité: 31.03.2000 FR 0004169
(43) Date de publication de la demande: 02.01.2003
(73) Titulaire: L'ORÉAL, 75008 Paris (FR)
(72) Inventeur: GIROUD, Franck, F-92110 Clichy (FR); SAMAIN, Henri, F-91570 Bièvres (FR); ROLLAT, Isabelle, F-75017 Paris (FR)
(74) Mandataire: Casalonga, Axel
(86) Numéro de dépôt international: PCT/FR2001/000952
(87) Numéro de publication internationale: WO 2001/074308

(56) Documents cités:
- EP-A- 0 159 628
- EP-A- 0 832 943
- EP-A- 1 064 918
- FR-A- 2 751 542
- FR-A- 2 779 637
- FR-A- 2 783 164
- US-A- 4 344 763
- US-A- 5 919 487
- PATENT ABSTRACTS OF JAPAN vol. 013, no. 171 (C-588), 24 avril 1989 (1989-04-24) & JP 64 000126 A (TORU YAMAMOTO;OTHERS: 01), 5 janvier 1989 (1989-01-05)
- PATENT ABSTRACTS OF JAPAN vol. 1996, no. 02, 29 février 1996 (1996-02-29) & JP 07 267640 A (IDEMITSU KOSAN CO LTD), 17 octobre 1995 (1995-10-17)

## Description

La présente invention concerne d'une manière générale des compositions cosmétiques aqueuses, en particulier pour le traitement des cheveux, comportant des nanoparticules et des composés organiques du silicium solubles dans l'eau.

Il existe des produits de coiffage dont l'utilisation permet d'augmenter le volume de la coiffure. Ces produits, à base de polymères filmogènes sont particulièrement appréciés par les utilisateurs portant des cheveux fins.

Ces produits de coiffage laissent un toucher peu naturel résultant du dépôt de polymère à la surface des cheveux.

Des compositions de coiffage des cheveux sont décrites par exemple dans US 4 344 763. Elles comprennent en solution dans l'isopropanol, au moins un aminoalkylalcoxysilane de formule R₁NH(CH₂)ₙSi(OR₂)₃, et un ester de type titanate liquide.

Il est possible, par ailleurs, d'utiliser des traitements permanents. Ces traitements basés sur l'utilisation de réducteur et d'oxydant, nécessitent une mise sous tension mécanique des cheveux via un matériel d'enroulage, pour donner une mise en forme durable de la chevelure. Ce procédé permet effectivement d'augmenter le volume de la chevelure. Cependant, ces traitements présentent l'inconvénient de modifier l'aspect de la chevelure (cheveux frisés) et de dégrader l'état des fibres.

Il n'existe pas de traitement permettant un accroissement du volume de la chevelure sans modification de la forme ou du toucher des cheveux.

Des composés organiques du silicium solubles dans l'eau sont connus du document FR 2 783 164 en combinaison avec une quantité d'un agent de neutralisation de sorte à ce que lesdits composés du silicium soient neutralisés à raison de 1/1000 à 99/100.

Des particules d'éléments métalliques, de métalloïdes, d'alliages métalliques, de carbures ou de nitrures d'éléments métalliques ou métalloïdes, en suspension dans un milieu cosmétiquement acceptable, sont connues de EP 1 064 918 pour procurer aux cheveux de la brillance.

On a maintenant découvert, de façon surprenante, qu'il était possible d'augmenter le volume de la chevelure sans modification de la forme ou du toucher des cheveux par l'emploi de compositions particulières décrites ci-dessous.

Ces compositions permettent d'augmenter le gonflant de la coiffure, sans altération du toucher des cheveux, ni de leur forme, sans dégradation de la fibre et sans adhésion entre les cheveux par une matière filmogène.

Par ailleurs, on s'est également aperçu que l'utilisation de ces compositions donnait au toucher une sensation de cheveux plus épais (bien que l'épaisseur des cheveux ne soit pas augmentée). Cette observation est particulièrement intéressante car de nombreux utilisateurs apprécient cette sensation de cheveux fortifiés.

Selon l'invention, les compositions sont caractérisées par le fait qu'elles contiennent, dans un milieu cosmétiquement acceptable comprenant de l'eau et/ou au moins un solvant, i) des nanoparticules choisies parmi les nanoparticules métalliques, d'oxydes métalliques, de carbures ou nitrures métalliques et leurs mélanges, et ii) un ou plusieurs composés organiques du silicium solubles dans le milieu cosmétiquement acceptable, choisis parmi les organosilanes comportant un, deux ou trois atomes de silicium et les organosiloxanes comportant deux ou trois atomes de silicium, les composés de silicium comportant en outre deux groupes hydrolysables ou hydroxyles par molécule, le rapport des concentrations "nanoparticules/composés organique du silicium variant de 0,05 à 2.

On entend par nanoparticules, des particules de taille inférieure à 200 nm et de préférence inférieure à 50 nm.

Parmi les nanoparticules d'oxyde métallique, on peut citer l'oxyde de cérium (CeO), l'alumine (Al₂O₃), l'oxyde de titane (TiO₂), les titanates (BaTiO₃, Ba_{0,5}Sr_{0,5}TiO_{3'} SrTiO₃), l'oxyde d'indium (In₂O₃), l'oxyde d'étain (SnO₂), l'oxyde d'antimoine (Sb₂O₃), l'oxyde de magnésium (MgO), l'oxyde de calcium (CaO), les oxydes de manganèse (Mn₃O₄, MnO₂), l'oxyde de molybdène (MoO₃), la silice (SiO₂), l'oxyde de zinc (ZnO), l'oxyde d'yttrium (Y₂O₃), etc, et leurs mélanges.

Parmi les nanoparticules métalliques, on peut citer les nanoparticules d'argent, d'or, de platine, d'indium, etc, et leurs mélanges.

Parmi les carbures et nitrures métalliques, on peut citer le carbure de silicium (SiC) et le nitrure de silicium (Si₃N₄).

De préférence, les nanoparticules sont des nanoparticules d'alumine.

La quantité de nanoparticules dans les compositions varie généralement de 0,01% à 30% en poids, de préférence de 0,05% à 5% en poids par rapport au poids total de la composition.

Le deuxième constituant essentiel des compositions de l'invention est le ou les composés de silicium, peu ou pas polymérisées, solubles dans le milieu cosmétiquement acceptable et plus particulièrement dans l'eau et/ou le ou les solvants de ce milieu.

Comme indiqué précédemment, les composés organiques du silicium sont choisis parmi les organosilanes solubles dans l'eau et/ou les solvants, comprenant un, deux ou trois atomes de silicium et les organosiloxanes solubles dans l'eau et/ou les solvants, comportant deux ou trois atomes de silicium, de préférence deux atomes de silicium, ces composés comportant en outre au moins deux groupes hydrolysables ou hydroxyles par molécule.

De préférence, les composés organiques du silicium de l'invention comportent trois groupes hydrolysables et/ou hydroxyles par molécule. Les groupes hydrolysables préférés sont choisis parmi les groupes alcoxy, aryloxy et halogène.

Le composé organique du silicium peut porter une ou plusieurs fonctions lui assurant une compatibilité avec l'eau et/ou les solvants, et/ou une affinité avec les fibres kératiniques.

De préférence, le composé organique de silicium porte une ou plusieurs fonctions solubilisantes, en particulier améliorant la solubilité dans l'eau, par exemple des fonctions basiques telles que des fonctions alkylamines, alkylpolyamines et des fonctions non basiques telles que des fonctions alkylalcools, alkylthiols, alkylacides, alkylpolyols, et alkyl polycarboxyliques.

Parmi les fonctions solubilisantes, les fonctions préférées sont les fonctions basiques, en particulier les fonctions amines primaires, secondaires et tertiaires.

La ou les fonctions chimiques basiques peuvent être partiellement ou totalement neutralisées, par exemple en ajoutant à la composition une quantité appropriée d'un acide tel que l'acide chlorhydrique, l'acide sulfurique ou les sels d'acide sulfurique, l'acide nitrique et les acides organiques mono-, di- ou tricarboxyliques.

Les acides préférés sont l'acide sulfurique et ses sels.

Les organosilanes préférés selon l'invention répondent à la formule : 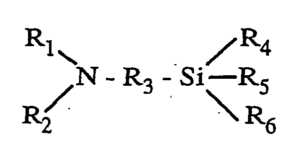 dans laquelle :
R₄ représente un halogène, un groupe OR' ou R'₁ ;
R₅ représente un halogène, un groupe OR" ou R'₂ ;
R₆ représente un halogène, un groupe OR'" ou R'₃ ;
et R¹, R², R³, R', R", R"', R'₁, R'₂, R'₃ représentent, indépendamment les uns des autres, un groupe hydrocarboné saturé ou insaturé, linéaire ou ramifié, portant éventuellement des groupes chimiques supplémentaires tels que des groupes acides ou amines, R₁, R₂, R', R" et R"' pouvant en outre désigner l'hydrogène, et
deux au moins des groupes R₄, R₅ et R₆ étant différents des groupes R'₁. R'₂ et R'₃.

De préférence, R₁, R₂, R', R" et R"', R'₁, R'₂ et R'₃ représentent un groupe alkyle en C₁₋₁₂, un groupe aryle en C₅₋₁₄, un groupe alkyl (C₁₋₈)-aryle (C₅₋₁₄), et un groupe aryl (C₅₋₁₄)-alkyle (C₁₋₈); et R₃ est de préférence un groupe alkylène en C₁₋₁₂, arylène en C₅₋₁₄, alkyl (C₁₋₈)-arylène (C₅-₁₄) et aryl (C₅₋₁₄)-alkyléne (C₁₋₈).

Les organosiloxanes préférés dans les compositions de la présente invention peuvent être représentés par la formule : 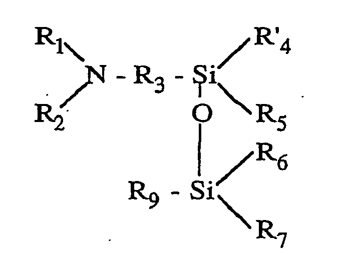 dans laquelle :
R₁, R₂, R₃, R₅ et R₆ sont définis comme précédemment;
R'₄ représente un halogène ou un groupe OR₁₁ ;
R₇ représente un halogène, un groupe OR₁₀ ou R"₁;
R₉ représente un halogène, un groupe ORg, R"₂ ou R₃NR₁R₂;
R"₁, R"₂, R₈, R₁₀ et R₁₁ représentent un groupe hydrocarboné, saturé ou insaturé, linéaire ou ramifié, portant éventuellement des groupes chimiques supplémentaires tels que des groupes solubilisants basiques;
R₁₁, R₁₀ et R₈ pouvant en outre désigner l'hydrogène.

De préférence R"₁, R"₂, R₈ ou R₁₀ et R₁₁ représentent un groupe alkyle en C₁₋₁₂, un groupe aryle en C₅₋₁₄, un groupe alkyl (C₁₋₈)-aryle (C₅₋₁₄), et un groupe aryl (C₅₋₁₄)-alkyle (C₁₋₈).

L'un au moins des groupes R₆, R₇ et R₉ désigne un halogène ou un groupe OR"', OR₁₀ ou OR₈.

De préférence, l'halogène est le chlore.

Les composés organiques du silicium préférés sont le 3-amino propyltriéthoxysilane, le 3-aminopropylméthyldiéthoxysilane et le 3-[bis(hydroxyéthyl)amino]propyltriéthoxysilane.

Le composé particulièrement préféré est le 3-aminopropyl triéthoxysilane.

La quantité de composés organique du silicium, présente dans la composition, représente en général 0,1 à 50%, de préférence 1 à 20% du poids total de la composition.

Le rapport de concentrations "nanoparticules/composé de silicium" est de 0,05 à 2.

Le taux des composés organiques du silicium selon l'invention est déterminé par des méthodes habituelles d'analyse telles que la spectroscopie RMN du silicium 29 et du proton, et par chromatographie.

Bien que les compositions selon l'invention soient de préférence des compositions aqueuses, on peut utiliser dans les compositions des solvants et mélanges de solvants tels qu'un alcool ou une cétone, par exemple l'alcool éthylique ou l'acétone.

De façon connue, toutes les compositions de l'invention peuvent contenir les adjuvants habituels dans le domaine cosmétique, tels que des huiles, cires ou autres corps gras usuels; des gélifiants et/ou épaississants classiques; des agents propénétrants; des agents réducteurs; des émulsionnants; des agents hydratants; des émollients, des filtres solaires; des agents adoucissants; des actifs hydrophiles ou lipophiles comme des céramides; des agents anti-mousse; des agents antiperspirants; des agents anti-radicaux libres; des tensio-actifs; des polymères fixants ou non; des protéines; des bactéricides; des séquestrants; des anti-pelliculaires; des anti-oxydants; des agents alcalinisants; des conservateurs; des parfums; des charges; des matières colorantes telles que des colorants et des pigments; des silicones volatiles ou non; des polyols; des protéines et des vitamines.

Les quantités de ces différents adjuvants sont celles classiquement utilisées dans le domaine considéré.

Bien entendu, l'homme de l'art veillera à choisir le ou les éventuels composés ajoutés à la composition selon l'invention de manière telle que les propriétés avantageuses attachées intrinsèquement à la composition conforme à l'invention ne soient pas, ou substantiellement pas, altérées par l'addition envisagée.

Les compositions selon l'invention sont de préférence utilisées en mode rincé, c'est-à-dire que l'on applique les compositions sur les cheveux, puis on les laisse poser quelques minutes ou plus avant de les rincer.

Les compositions selon l'invention peuvent se présenter sous toutes les formes appropriées pour une application topique, notamment sous forme de solutions du type lotions ou sérum; sous forme de gels aqueux; sous forme d'émulsions obtenues par dispersion d'une phase grasse dans une phase aqueuse (H/E) ou inversement (E/H), de consistance liquide plus ou moins épaisse telle que des laits et des crèmes plus ou moins onctueuses.

Ces compositions sont préparées selon les méthodes usuelles.

Les compositions selon l'invention sont de préférence utilisées comme produits capillaires, notamment pour le maintien de la coiffure ou de la mise en forme des cheveux. Elles peuvent en outre apporter de la coloration temporaire aux cheveux, bien assurer la protection des cheveux contre les effets des radiation UV, tout en apportant des propriétés de maintien ou de fixation des cheveux.

Les compositions capillaires, selon l'invention, sont de préférence des produits de coiffage tels que des shampooings, des gels, des lotions de mises en pli, des lotions pour le brushing, des compositions de fixation et de coiffage telles que des laques ou spray.

Les lotions peuvent être conditionnées sous diverses formes, notamment dans des vaporisateurs, des flacons-pompe ou dans des récipients aérosol afin d'assurer une application de la composition sous forme vaporisée ou sous forme de mousse. De telles formes de conditionnement sont indiquées, par exemple lorsqu'on souhaite obtenir un spray, une mousse pour la fixation ou le traitement des cheveux.

Les nanoparticules peuvent également être introduites dans la composition directement au moment de l'emploi.

La présente invention a également pour objet l'utilisation de la composition selon l'invention dans un procédé de traitement des cheveux.

Selon un mode de réalisation de ce procédé, on applique la composition sur les cheveux rincés ou non, de préférence sous la forme d'un spray, soit à l'aide d'un flacon pompe, soit à l'aide d'un aérosol.

Après pulvérisation sur l'ensemble de la chevelure, on laisse agir et sécher la composition.

Les cheveux sont alors rincés à l'eau.

Les cheveux peuvent être mis dans la forme souhaitée, soit avant l'application, soit immédiatement après.

Le temps de séchage peut être variable et est fonction de la nature de la composition.

Les cheveux, après peignage, présentent un meilleur gonflant et une qualité de toucher très agréable.

L'invention est illustrée par les exemples suivants :

### EXEMPLE 1

On a réalisé les quatre formulations suivantes :

| Formulation | Aminopropyltriéthoxysilane | Nanoparticules d'alumine* | Eau |
|---|---|---|---|
| | (% en poids) | (% en poids) | (% en poids) |
| 1 | 12,5 | 0,5 | 87 |
| Comparative A | 12,5 | - | 87,5 |
| Comparative B | - | 0,5 | 99,5 |
| Témoin | - | - | 100 |

| | | | |
|---|---|---|---|
| * - Nanoparticules commercialisées sous la dénomination "NANOTEK^{®} aluminium oxyde de dispersion" par la Société NANOPHASE TECHNOLOGIES CORPORATION. | | | |

5 g de la formulation 1 décrite ci-dessus et 5 g de la formulation comparative A sont appliqués sur deux chevelures différentes (cheveux européens châtains de 20 cm de long environ) par pulvérisation à l'aide d'un spray. Après 15 minutes de pose, la chevelure est rincée à l'eau.

Afin de montrer l'effet de gonflant du cheveu engendré par l'application des diverses formulations précédentes, on a mesuré à l'aide de la méthode dite du "pendule de souplesse" l'évolution de la rigidité en flexion des cheveux après traitement des fibres.

Le pendule utilisé est du type balançoire rigide qui bat la seconde. Ce pendule est constitué d'une barre de flexion en laiton poli. Cette barre est reliée à un axe par une tige de longueur L=30 cm. Sur cette tige, à une distance de L'=18,5 cm de l'axe de rotation, est fixée une barre pesante de masse m=47 g. L'énergie potentielle initiale du balancier est fixée par son angle d'inclinaison noté θ.

Les éprouvettes de cheveux se présentent sous la forme d'un peigne de 39 fragments de cheveux de 11 mm de long collés parallèlement sur un support métallique.

La mesure s'effectue de la façon suivante : la barre en laiton est lâchée d'un angle θ=30° sans vitesse initiale. A chaque passage au point bas (θ=0), la barre vient fléchir l'éprouvette de 39 cheveux et perd une part de son énergie potentielle jusqu'à son arrêt complet. Plus l'arrêt du pendule est rapide, plus les cheveux sont rigides. On considère que la perte d'énergie liée au frottement de la barre de laiton sur les cheveux est négligeable.

Pour mettre en évidence l'effet de gonflant des cheveux, on effectue une première mesure avant traitement puis une seconde mesure après traitement, puis on compare le nombre de battements nécessaire à l'arrêt du pendule avant et après traitement.

Le traitement des cheveux se réalise directement sur les cheveux montés sur les éprouvettes afin d'obtenir une plus grande sensibilité de la mesure. Pour chaque traitement, on réalise une série de mesures sur 10 éprouvettes. Les mesures ainsi que le séchage des éprouvettes de cheveux s'effectuent à température et humidité relative contrôlées (20°C et 45%

Dans les conditions d'application précédemment indiquées, on note que les différents traitements du cheveu induisent une diminution du nombre de battements nécessaire à l'arrêt du pendule.

Les différents pourcentages de variation du nombre de battements du pendule après application des formulations précédemment décrites, sont regroupés dans le tableau ci-dessous.

| Traitement | Formulation 1 | Formulation comparative A | Formulation comparative B |
|---|---|---|---|
| % de variation du nombre de battements du pendule après traitement | - 15% ± 2% | - 6% ± 1% | - 10% ± 2% |

Ce résultat montre que l'application de nanoparticules d'alumine permet de rigidifier le cheveu. Ceci explique le moindre fléchissement des fibres observé sous l'effet de la pesanteur, visualisé par un gonflant plus net de la chevelure. On note également que l'effet de gonflant de la fibre est accru lorsque les nanoparticules sont associées à un composé (formulation comparative B) du type 3-aminopropyltriéthoxysilane.

On note un effet net de moindre fléchissement des fibres sous l'effet de la pesanteur lors de l'utilisation de la formulation 1 vis-à-vis de la formulation comparative A. Ce phénomène se traduit visuellement par un gonflant plus marqué de la coiffure après l'application de cette formulation. Cette rigidification de la fibre est amplifiée lors de l'utilisation de l'organosilane. En effet, la formulation 1 donne un volume plus important à la chevelure que la formulation comparative B, elle-même plus efficace que le témoin.

## Revendications

1. Composition cosmétique comprenant, dans un milieu cosmétiquement acceptable incluant de l'eau et/ou un ou plusieurs solvants, (i) des nanoparticules choisies parmi les nanoparticules métalliques, d'oxydes métalliques, de carbures métalliques, de nitrures métalliques et leurs mélanges, et (ii) au moins un composé organique du silicium soluble dans le milieu cosmétiquement acceptable choisi parmi les organosilanes comprenant un, deux ou trois atomes de silicium, et les organosiloxanes comprenant deux ou trois atomes de silicium, ces composés organiques du silicium comportant en outre au moins deux groupes hydroxyles ou hydrolysables par molécule, le rapport des concentrations "nanoparticules/composé organique du silicium variant de 0,05 à 2.

2. Composition cosmétique selon la revendication 1, **caractérisée en ce que** les composés organiques du silicium comportent trois groupes hydrolysables ou hydroxyles par molécule.

3. Composition selon la revendication 1 ou 2, **caractérisée en ce que** le composé organique du silicium comporte en outre au moins un groupe à fonction solubilisante.

4. Composition selon la revendication 3, **caractérisée en ce que** le groupe à fonction solubilisante est un groupe à fonction basique.

5. Composition selon la revendication 4, **caractérisée en ce que** la fonction basique est choisie parmi les amines primaires, secondaires ou tertiaires.

6. Composition selon la revendication 4 ou 5, **caractérisée en ce que** la fonction basique est partiellement ou totalement neutralisée.

7. Composition selon l'une quelconque des revendications précédentes, **caractérisée en ce que** les groupes hydrolysables sont choisis parmi les groupes alcoxy, aryloxy et halogène.

8. Composition cosmétique selon l'une quelconque des revendications précédentes, **caractérisée en ce que** le ou les composés organiques du silicium sont choisis parmi les composés de formules : 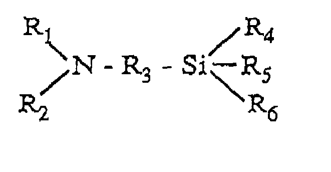 dans laquelle:
R₄ représente un halogène, un groupe OR' ou R'₁;
R₅ représente un halogène, un groupe OR" ou R'₂ ;
R₆ représente un halogène, un groupe OR'" ou R'₃ ;
R₁, R₂, R₃, R', R", R"', R'₁, R'₂, R'₃ représentent, indépendamment les uns des autres, un groupe hydrocarboné saturé ou insaturé, linéaire ou ramifié, portant éventuellement des groupes chimiques supplémentaires, R₁, R₂, R', R" et R"' pouvant en outre désigner l'hydrogène, deux au moins des groupes R₄, R₅ et R₆ étant différents des groupes R'₁, R'₂ et R'₃ ; et 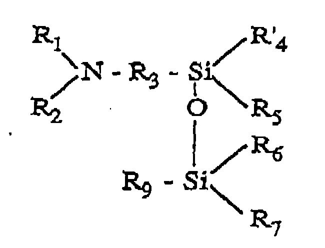
dans laquelle :
R₁, R₂, R₃, R₅ et R₆ sont définis comme précédemment;
R'₄ représente un halogène ou un groupe OR₁₁;
R₇ représente un halogène, un groupe OR₁₀ ou R"₁;
R₉ représente un halogène, un groupe OR₈, R"₂ ou R₃NR₁R₂;
R"₁, R"₂, R₈, R₁₀ et R₁₁ représentent un groupe hydrocarboné, saturé ou insaturé, linéaire ou ramifié, portant éventuellement des groupes chimiques supplémentaires, les groupes R₁₁, R₁₀ et R₈ pouvant en outre désigner l'hydrogène; l'un au moins des groupes R₆, R₇ et R₉ désignant un halogène, un groupe OR"', OR₁₀ ou OR₈.

9. Composition cosmétique selon la revendication 8, **caractérisée en ce que** les groupes R₁, R₂, R', R'₁ R'₂, R'₃, R", R"' R"₁, R"₂, R₈, et R₁₁ sont choisis parmi les radicaux alkyle en C₁₋₁₂, aryle en C₅₋₁₄, alkyl (C₁₋₈)-aryle (C₅₋₁₄), et aryl (C₅₋₁₄)-alkyle (C₁₋₈).

10. Composition selon l'une quelconque des revendications précédentes, **caractérisée en ce que** le ou les composés organiques du silicium représentent 0,1 à 50%, de préférence 1 à 20%, du poids total de la compositions.

11. Composition selon l'une quelconque des revendications précédentes, **caractérisée en ce que** les nanoparticules d'oxydes métalliques sont choisies parmi CeO, Al₂O₃, TiO₂, BaTiO₃, Ba_{0,5}Sr_{0,5} TiO₃, SrTiO₃, In₂O₃, SnO₂, Sb₂O₃, MgO, CaO, Mn₃O₄, MnO₂, MoO₃, SiO₂, ZnO, Y₂O₃, et leurs mélanges.

12. Composition selon l'une quelconque des revendications 1 à 10, **caractérisée en ce que** les nanoparticules métalliques sont choisies parmi Ag, Au, Pt, In et leurs mélanges.

13. Composition selon l'une quelconque des revendications précédentes, **caractérisée en ce que** les nanoparticules ont une dimension inférieure à 200 nm, de préférence inférieure à 50 nm.

14. Composition selon l'une quelconque des revendications précédentes, **caractérisée en ce que** les nanoparticules représentent 0,01 à 30%, de préférence 0,05 à 5% du poids total de la composition.

15. Composition selon l'une quelconque des revendications précédentes, **caractérisée par le fait qu'**il s'agit d'un produit capillaire.

16. Composition selon la revendication 15, **caractérisée par le fait qu'**il s'agit d'un produit capillaire pour le maintien de la coiffure ou la mise en forme des cheveux.

## Claims

1. Cosmetic composition comprising, in a cosmetically acceptable medium including water and/or one or more solvents, i) nanoparticles chosen from metallic nanoparticles, of metal oxides, of metal carbides, of metal nitrides and mixtures thereof, and ii) at least one organosilicon compound which is soluble in the cosmetically acceptable medium and is chosen from organosilanes comprising one, two or three silicon atoms and organosiloxanes comprising two or three silicon atoms, these organosilicon compounds also comprising at least two hydroxyl or hydrolyzable groups per molecule, the "nanoparticles/organosilicon compound" concentration ratio ranging from 0.05 to 2.

2. Cosmetic composition according to Claim 1, **characterized in that** the organosilicon compounds comprise three hydrolyzable or hydroxyl groups per molecule.

3. Composition according to Claim 1 or 2, **characterized in that** the organosilicon compound also comprises at least one group containing a solubilizing function.

4. Composition according to Claim 3, **characterized in that** the group containing a solubilizing function is a group containing a basic function.

5. Composition according to Claim 4, **characterized in that** the basic function is chosen from primary, secondary and tertiary amines.

6. Composition according to Claim 4 or 5, **characterized in that** the basic function is partially or totally neutralized.

7. Composition according to any one of the preceding claims, **characterized in that** the hydrolyzable groups are chosen from alkoxy, aryloxy and halogen groups.

8. Cosmetic composition according to any one of the preceding claims, **characterized in that** the organosilicon compound(s) is (are) chosen from the compounds of formulae: 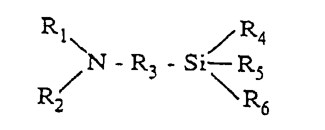 in which:
R₄ represents a halogen or a group OR' or R'₁;
R₅ represents a halogen or a group OR" or R'₂;
R₆ represents a halogen or a group OR"' or R'₃;
and R₁, R₂, R₃ , R', R " , R "' , R'₁, R'₂ and R'₃ represent, independently of each other, a saturated or unsaturated, linear or branched hydrocarbon-based group optionally bearing additional chemical groups, R₁, R₂, R', R" and R"' also possibly denoting hydrogen, at least two of the groups R₄, R₅ and R₆ being other than groups R'₁, R'₂ and R'₃; and 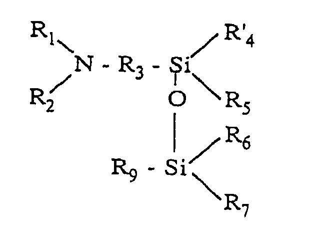
in which:
R₁, R₂ , R₃ , R₅ and R₆ are defined as above;
R'₄ represents a halogen or a group OR₁₁;
R₇ represents a halogen or a group OR₁₀ or R"₁;
R₉ represents a halogen or a group OR₈, R"₂ or R₃NR₁R₂;
R"₁, R"_{2,} R₈, R₁₀ and R₁₁ represent a saturated or unsaturated, linear or branched hydrocarbon-based group optionally bearing additional chemical groups, the groups R₁₁, R₁₀ and R₈ also possibly denoting hydrogen; at least one of the groups R₆, R₇ and R₉ denoting a halogen or a group OR"', OR₁₀ or OR₈.

9. Cosmetic composition according to Claim 8, **characterized in that** the groups R₁, R₂, R', R'₁, R'₂, R'₃, R", R"' , R"₁, R"₂, R₈ , R₁₀ and R₁₁ are chosen from C₁₋₁₂alkyl, C₅₋₁₄aryl, (C₁₋₈) alkyl (C₅₋₁₄) aryl and (C₅-C₁₄) aryl (C₁₋₈) alkyl radicals.

10. Composition according to any one of the preceding claims, **characterized in that** the organosilicon compound(s) represent(s) 0.1% to 50% and preferably 1% to 20% of the total weight of the composition.

11. Composition according to any one of the preceding claims, **characterized in that** the metal oxide nanoparticles are chosen from CeO, Al₂O₃, TiO₂, BaTiO₃, Ba_{0.5}Sr_{0.5}TiO₃, SrTiO₃ , In₂O₃ , SnO₂ , Sb₂O₃ , MgO, CaO, Mn₃O₄, MnO₂, MoO₃, SiO₂, ZnO and Y₂O₃, and mixtures thereof.

12. Composition according to any one of Claims 1 to 10, **characterized in that** the metal nanoparticles are chosen from Ag, Au, Pt and In, and mixtures thereof.

13. Composition according to any one of the preceding claims, **characterized in that** the nanoparticles are less than 200 nm and preferably less than 50 nm in size.

14. Composition according to any one of the preceding claims, **characterized in that** the nanoparticles represent 0.01% to 30% and preferably 0.05% to 5% of the total weight of the composition.

15. Composition according to any one of the preceding claims, **characterized in that** it is a hair product.

16. Composition according to Claim 15, **characterized in that** it is a hair product for holding the hairstyle or shaping the hair.

## Patentansprüche

1. Kosmetische Zusammensetzung umfassend in einem kosmetisch annehmbaren Milieu beinhaltend Wasser und/oder ein oder mehrere Lösungsmittel, (i) Nanopartikel, ausgewählt aus den metallischen Nanopartikeln, metallischen Oxyden, Metallkarbiden, Metallnitriden und ihre Mischungen, und (ii) wenigstens eine organische, im kosmetisch annehmbaren Milieu lösliche Siliziumverbindung, ausgewählt aus den Organosilanen umfassend ein, zwei oder drei Siliziumatome, und den organosiloxanen umfassend zwei oder drei Siliziumatome, wobei diese organischen Siliziumverbindungen des Weiteren pro Molekül wenigstens zwei Hydroxylgruppen oder hydrolysierbare Gruppen aufweisen und wobei das Verhältnis der Konzentrationen "Nanopartikel/organische Siliziumverbindung" zwischen 0,05 und 2 variiert.

2. Kosmetische Zusammensetzung gemäß Anspruch 1, **dadurch gekennzeichnet, dass** die organischen Siliziumverbindungen pro Molekül drei hydrolysierbare Gruppen oder Hydroxylgruppen aufweisen.

3. Kosmetische Zusammensetzung gemäß Anspruch 1 oder 2, **dadurch gekennzeichnet, dass** die organische Siliziumverbindung des Weiteren wenigstens eine Gruppe mit solubilisierender Funktion aufweist.

4. Kosmetische Zusammensetzung gemäß Anspruch 3, **dadurch gekennzeichnet, dass** die Gruppe mit solubilisierender Funktion eine Gruppe mit basischer Funktion ist.

5. Kosmetische Zusammensetzung gemäß Anspruch 4, **dadurch gekennzeichnet, dass** die basische Funktion aus den primären, sekundären oder tertiären Aminen ausgewählt ist.

6. Kosmetische Zusammensetzung gemäß Anspruch 4 oder 5, **dadurch gekennzeichnet, dass** die basische Funktion teilweise oder vollständig neutralisiert ist.

7. Kosmetische Zusammensetzung nach einem der vorhergehenden Ansprüche, **dadurch gekennzeichnet, dass** die hydrolisierbaren Gruppen aus den Alcoxy-, Aryloxy- und Halogengruppen ausgewählt sind.

8. Kosmetische Zusammensetzung nach einem der vorhergehenden Ansprüche, **dadurch gekennzeichnet, dass** die organische/n Siliziumverbindung/en ausgewählt werden aus den Verbindungen gemäß Formeln: 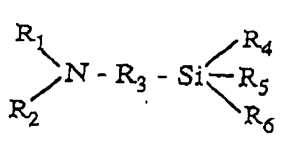 in welcher:
R₄ ein Halogen, eine Gruppe OR' oder R'₁ darstellt;
R₅ ein Halogen, eine Gruppe OR" oder R'₂ darstellt;
R₆ ein Halogen, eine Gruppe OR''' oder R'₃ darstellt;
R₁, R₂, R₃ , R', R", R"', R'₁, R'₂, R'₃ unabhängig von einander eine gesättigte oder ungesättigte, lineare oder verzweigte Kohlenwasserstoffgruppe darstellen, welche gegebenenfalls zusätzliche chemische Gruppen aufweisen, des Weiteren R₁, R₂, R', R " , R''' Wasserstoff darstellen können, wobei zwei von wenigstens der Gruppen R₄, R₅ und R₆ unterschiedlich zu den Gruppen R'₁, R'₂ und R'₃ sind; und 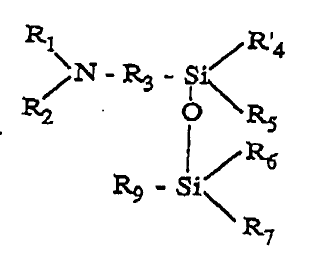
in welcher:
R₁, R₂, R₃, R₅ und R₆ wie vorhergehend definiert sind;
R'₄ ein Halogen oder eine Gruppe OR₁₁ darstellt;
R₇ ein Halogen, eine Gruppe OR₁₀ oder R"₁ darstellt;
R₉ ein Halogen, eine Gruppe OR₈, R''₂ oder R₃NR₁R₂ darstellt;
R''_{1,} R''₂, R₈, R₁₀ und R₁₁ eine gesättigte oder ungesättigte, lineare oder verzweigte Kohlenwasserstoffgruppe darstellen, welche gegebenenfalls zusätzliche chemische Gruppen aufweisen, des Weiteren die Gruppen R₁₁, R₁₀ und R₈ Wasserstoff darstellen können; wobei eine von wenigstens der Gruppen R₆, R₇ und R₉ ein Halogen, eine Gruppe OR"', OR₁₀ oder OR₈ darstellen.

9. Kosmetische Zusammensetzung gemäß Anspruch 8, **dadurch gekennzeichnet, dass** die Gruppen R₁, R₂, R', R'₁, R'₂, R'₃, R" , R"' , R''1, R''2, R₈, R₁₀ und R₁₁ aus den Resten C₁₋₁₂ Alkyl, C₅₋₁₄ Aryl, C₁₋₈ Alkyl- C₅₋₁₄ Aryl und C₅₋₁₄ Aryl- C₁₋₈ Alkyl ausgewählt sind.

10. Kosmetische Zusammensetzung nach einem der vorhergehenden Ansprüche, **dadurch gekennzeichnet, dass** die organische/n Siliziumverbindung/en 0,1 bis 50 %, vorzugsweise 1 bis 20 %, des Gesamtgewichts der Zusammensetzung ausmachen.

11. Kosmetische Zusammensetzung nach einem der vorhergehenden Ansprüche, **dadurch gekennzeichnet, dass** die Metalloyxd-Nanopartikel aus CeO, Al₂O₃, TiO₂, BaTiO₃, Ba_{0,5}Sr_{0,5}TiO₃, SrTiO₃, In₂O₃, SnO₂, Sb₂O₃, MgO, CaO, Mn₃O₄, MnO₂, MoO₃, SiO₂, ZnO, Y₂O₃ und ihren Mischungen ausgewählt sind.

12. Kosmetische Zusammensetzung nach einem der Ansprüche 1 bis 10, **dadurch gekennzeichnet, dass** die metallischen Nanopartikel aus Ag, Au, Pt, In und ihren Mischungen ausgewählt sind.

13. Kosmetische Zusammensetzung nach einem der vorhergehenden Ansprüche, **dadurch gekennzeichnet, dass** die Nanopartikel eine Größe von kleiner als 200 nm, vorzugsweise kleiner als 50 nm besitzen.

14. Kosmetische Zusammensetzung nach einem der vorhergehenden Ansprüche, **dadurch gekennzeichnet, dass** die Nanopartikel 0,01 bis 30 %, vorzugsweise 0,05 bis 5 %, des Gesamtgewichts der Zusammensetzung ausmachen.

15. Kosmetische Zusammensetzung nach einem der vorhergehenden Ansprüche, **dadurch gekennzeichnet, dass** es sich um ein Haarpflegemittel handelt.

16. Kosmetische Zusammensetzung nach Anspruch 15, **dadurch gekennzeichnet, dass** es sich um ein Haarpflegemittel zum Erhalt der Frisur oder zum In-Form-Bringen der Haare handelt.
